# EUROPÄISCHE PATENTANMELDUNG

(11) **EP 3 282 376 A1**
(43) Veröffentlichungstag der Anmeldung: **14.02.2018**
(21) Anmeldenummer: 16183821.4
(22) Anmeldetag: 11.08.2016
(51) Int. Cl.: G06F 19/00

(54) **SYSTEM UND VERFAHREN ZUM ABGLEICH VON SYSTEMKONFIGURATIONEN FÜR MAGNETRESONANZTOMOGRAPHEN**

(71) Anmelder: Siemens Healthcare GmbH, 91052 Erlangen (DE)
(72) Erfinder: Schor, Stefan, 91054 Erlangen (DE)

(57) **Zusammenfassung**

Die Erfindung betrifft ein Verfahren zum Abgleich einer Systemkonfiguration eines Magnetresonanztomographen sowie ein System und einen Magnetresonanztomographen zur Ausführung des Verfahrens. Der Magnetresonanztomograph weist eine Steuerung und eine Ausgabeeinheit und die Konfigurationsvorrichtung eine Steuerung und eine Eingabeeinheit auf. Ein Datenspeicher steht über eine erste Datenverbindung mit dem Magnetresonanztomographen und über eine zweite Datenverbindung mit der Konfigurationsvorrichtung in Datenverbindung. In einem Schritt wird eine erste Systemkonfiguration des Magnetresonanztomographen auf dem Datenspeicher gespeichert. In einem anderen Schritt erzeugt der Magnetresonanztomograph eine Identifikation für die erste Systemkonfiguration und gibt sie in einem weiteren Schritt aus. In einem anderen Schritt erfasst die Konfigurationsvorrichtung die Identifikation mit der Eingabeeinheit und greift mittels der Identifikation auf die erste Systemkonfiguration in dem Datenspeicher zu.

## Beschreibung

Die Erfindung betrifft ein Verfahren sowie ein System zum Abgleich von Systemkonfigurationen für einen Magnetresonanztomographen. Das System weist einen Magnetresonanztomographen, eine Konfigurationsvorrichtung mit einer Steuerung, eine Eingabeeinheit und einen Datenspeicher für Systemkonfigurationen unterschiedlicher Magnetresonanztomographen auf.

Magnetresonanztomographen sind bildgebende Vorrichtungen, die zur Abbildung eines Untersuchungsobjektes Kernspins des Untersuchungsobjektes mit einem starken äußeren Magnetfeld ausrichten und durch ein magnetisches Wechselfeld zur Präzession um diese Ausrichtung anregen. Die Präzession bzw. Rückkehr der Spins aus diesem angeregten in einen Zustand mit geringerer Energie wiederum erzeugt als Antwort ein magnetisches Wechselfeld, auch als Magnetresonanzsignal bezeichnet, das über Antennen empfangen wird.

Mit Hilfe von magnetischen Gradientenfeldern wird den Signalen eine Ortskodierung aufgeprägt, die nachfolgend eine Zuordnung von dem empfangenen Signal zu einem Volumenelement ermöglicht. Das empfangene Signal wird dann ausgewertet und eine dreidimensionale bildgebende Darstellung des Untersuchungsobjektes bereitgestellt. Die erzeugte Darstellung gibt eine räumliche Dichteverteilung der Spins an.

Untersuchungen mit Magnetresonanztomographen können durch Konfigurationsdateien automatisiert werden, die die Abläufe der einzelnen Komponenten des Magnetresonanztomographen bei der Untersuchung beschreiben und steuern. Selbst bei dem gleichen Magnetresonanztomographen eines Herstellers gibt es unterschiedliche Ausstattungsvarianten der Hardware, beispielsweise unterschiedliche Anzahl an Empfangs- und Sendekanälen oder unterschiedliche Lokalspulen, und unterschiedliche Softwaremodule.

Magnetresonanztomographen sollen aus Gründen der Kosteneffizienz möglichst durch Untersuchungen ausgelastet sein und nicht durch das Ausarbeiten und Eingeben von Konfigurationen und Untersuchungsabläufen. Die Konfigurationsdateien werden daher vorzugsweise auf Konfigurationsvorrichtungen, beispielsweise Computern oder Workstations mit spezieller Software zusammengestellt. Aufgrund der unterschiedlichen Hard- und Softwareausstattungen kommt es jedoch immer wieder vor, dass eine Konfigurationsdatei aufgrund der unterschiedlichen Ausstattungsvarianten beim Einspielen in einen Magnetresonanztomographen dann doch nicht lauffähig ist und teure Systemzeit zur Anpassung oder Konvertierung erfordert.

Es ist daher eine Aufgabe der vorliegenden Erfindung, das Konfigurieren eines Magnetresonanztomographen schneller, sicherer und kostengünstiger zu machen.

Die Aufgabe wird durch einen erfindungsgemäßen Magnetresonanztomographen des Anspruchs 1, durch ein erfindungsgemäßes System nach Anspruch 2 und ein erfindungsgemäßes Verfahren nach Anspruch 5 gelöst.

Das erfindungsgemäße Verfahren ist zum Abgleich einer ersten Systemkonfiguration eines Magnetresonanztomographen mit einer Konfigurationsvorrichtung mit Hilfe eines Datenspeichers vorgesehen. Als Systemkonfiguration wird im Sinne der Erfindung eine Sammlung an Information angesehen, die alle Parameter des Magnetresonanztomographen zusammenfasst bzw. beschreibt, die Einfluss auf die Ausführung von Konfigurationsdateien haben. Dies können beispielsweise Softwareversion, Softwaremodule, Hardwareausstattung oder auch Zusatzgeräte sein. Der Datenspeicher ist ausgelegt, eine Mehrzahl an Systemkonfigurationen von einer Mehrzahl an Magnetresonanztomographen zu speichern.

Zur Ausführung des Verfahrens weist der Magnetresonanztomograph eine Steuerung und eine Ausgabeeinheit auf. Die Konfigurationsvorrichtung weist eine Steuerung und eine Eingabeeinheit auf. Dabei sind die Ausgabeeinheit und die Eingabeeinheit insofern kompatibel zueinander, dass eine von der Ausgabeeinheit des Magnetresonanztomographen ausgegebene Information von der Eingabeeinheit Konfigurationsvorrichtung erfasst werden kann.

Zwischen Magnetresonanztomograph und Datenspeicher besteht eine erste Datenverbindung und zwischen Konfigurationsvorrichtung und Datenspeicher eine zweite Datenverbindung, sodass die Konfigurationsvorrichtung und der Magnetresonanztomograph Zugriff auf den Datenspeicher haben. Die erste bzw. zweite Datenverbindung können beispielsweise ein lokales Netz oder das Internet sein, oder auch Punkt-zu-Punkt-Datenverbindungen.

In einem Schritt des erfindungsgemäßen Verfahrens speichert die Steuerung des Magnetresonanztomographen eine erste Systemkonfiguration des Magnetresonanztomographen auf dem Datenspeicher.

In einem anderen Schritt des erfindungsgemäßen Verfahrens erzeugt die Steuerung eine Identifikation der ersten Systemkonfiguration. Eine Identifikation im Sinne der Erfindung erlaubt eine eindeutige Zuordnung der ersten Systemkonfiguration zu dem Magnetresonanztomographen. Darüber hinaus kann die Identifikation auch einen Zugriff auf die erste Systemkonfiguration auf dem Datenspeicher ermöglichen. Die Identifikation könnte beispielsweise auch einen Pfad oder Link aufweisen.

In einem weiteren Schritt gibt der Magnetresonanztomograph die Identifikation über die Ausgabeeinheit aus. Unterschiedliche Optionen der Ausgabe sind in Unteransprüchen angegeben.

In einem weiteren Schritt des erfindungsgemäßen Verfahrens erfasst die die Eingabeeinheit der Konfigurationsvorrichtung die Identifikation.

In einem weiteren Schritt des erfindungsgemäßen Verfahrens greift die Konfigurationsvorrichtung über die Datenverbindung mithilfe der Identifikation auf die erste Systemkonfiguration in dem Datenspeicher zu. Beispielsweise lädt die Konfigurationseinheit die erste Systemkonfiguration oder Teile davon in einen internen Speicher, um Informationen aus der ersten Systemkonfiguration für die Erstellung einer Ablaufkonfiguration zu nutzen.

Das erfindungsgemäße Verfahren ermöglicht auf vorteilhafte Weise sicherzustellen, dass bei der Erstellung einer Ablaufkonfiguration für einen Magnetresonanztomographen die korrekte Systemkonfiguration zugrunde gelegt wird und so unnötige Nacharbeiten vermieden werden. Gleichzeitig wird eine doppelte Datenhaltung vermieden und ein unzulässiger Zugriff auf die Systeme erschwert.

Der erfindungsgemäße Magnetresonanztomograph weist eine Steuerung und eine Ausgabeeinheit auf. Die Steuerung des Magnetresonanztomographen ist ausgelegt, eine erste Systemkonfiguration in einem Datenspeicher für eine Mehrzahl an Systemkonfigurationen über eine Datenverbindung abzuspeichern. Weiterhin ist die Steuerung des Magnetresonanztomographen ausgelegt, eine Identifikation für die erste Systemkonfiguration vorzugsweise auf einem maschinenlesbaren Medium mittels der Ausgabeeinheit auszugeben.

Die Identifikation der Konfiguration, vorzugsweise auf einem maschinenlesbaren Medium, erleichtert auf vorteilhafte Weise den Zugriff auf die gespeicherte erste Systemkonfiguration und verhindert Fehler durch eine manuelle Weitergabe der Daten.

Das erfindungsgemäße System aus einem erfindungsgemäßen Magnetresonanztomographen und einer Konfigurationsvorrichtung mit einer Steuerung und einer Eingabeeinheit teilt die Vorteile des erfindungsgemäßen Magnetresonanztomographen.

Weitere vorteilhafte Ausführungsformen sind in den abhängigen Ansprüchen angegeben.

In einer möglichen Ausführungsform des erfindungsgemäßen Systems ist die Konfigurationsvorrichtung ausgelegt, anhand der Identifikation die erste Systemkonfiguration in dem Datenspeicher zu identifizieren. Beispielsweise kann die Identifikation einen Link oder einen Pfadnamen in dem Datenspeicher angeben. Es ist aber ebenso denkbar, dass die Identifikation einen Schlüssel oder anonymisierte Bezeichnung enthält, um die Systemkonfigurationen zu schützen. Die Konfigurationsvorrichtung ist ausgelegt, in Abhängigkeit von der ersten Systemkonfiguration eine Ablaufkonfiguration für den Magnetresonanztomographen bereitzustellen, der die Identifikation ausgegeben hat. Beispielsweise kann die Konfigurationseinheit mittels der Identifikation die erste Systemkonfiguration in einen internen Speicher laden und zur Erstellung einer Konfigurationsdatei nutzen. Es ist aber auch denkbar, dass die Konfigurationsvorrichtung mittels der Identifikation einzelne Parameter aus dem Datenspeicher abfragt.

Indem die Konfigurationsvorrichtung Zugriff auf die korrekte Systeminformation hat, kann die Kompatibilität der erzeugten Konfigurationsdatei mit dem Magnetresonanztomographen auf vorteilhafte Weise sichergestellt werden.

In einer denkbaren Ausführungsform des erfindungsgemäßen Systems ist die Ausgabeeinheit ausgelegt, einen QR-Code mit der Identifikation bereitzustellen und die Eingabeeinheit ist ausgelegt, den QR-Code zu erfassen. In einer weiteren Ausführungsform ist auch ein Barcode anstelle des QR-Codes möglich. Optische Codes können auch auf einem Display ausgegeben und durch eine Kamera oder Scanner als Eingabeeinheit der Konfigurationsvorrichtung erfasst werden. Vorstellbar wären auch andere maschinenschreib- und lesbare Medien wie Speicherchips, magnetisch Speicher, optische Speicher oder andere. Die Medien können auch mit kryptologischen Methoden gegen ein Auslesen durch nicht autorisierte Personen oder Geräte gesichert sein.

Die maschinenlesbaren Medien und insbesondere der QR-Code erlauben eine zuverlässige und sichere Übertragung der Identifikation mit einfachen Mitteln. Der QR-Code oder ein Bar-Code schließen darüber hinaus aus, dass weitere Informationen von dem Magnetresonanztomographen unerlaubt abgezogen werden oder beispielsweise Computerviren in den Magnetresonanztomographen gelangen.

In einer möglichen Ausführungsform des erfindungsgemäßen Verfahrens weist das Verfahren weiterhin den Schritt auf, eine Ablaufkonfiguration für den Magnetresonanztomographen in Abhängigkeit von der ersten Systemkonfiguration bereitzustellen.

Unter Ablaufkonfiguration ist dabei eine Folge von Schritten zu verstehen, die der Magnetresonanztomograph zur Aufnahme einer MR-Abbildung auszuführen hat. Die Ablaufkonfigurationen können sich beispielsweise darin unterscheiden, wie schnell eine Aufnahme erfolgen soll und entsprechende schnelle Sequenzen aufweisen. Weitere Unterschiede können beispielsweise die Auflösung, den Kontrast, den zu erfassenden Teil des Patienten oder zu erfassende Stoffe betreffen. Denkbar ist es auch, dass die Ablaufkonfigurationen sich beispielsweise in Parametern unterscheiden, die den Patienten betreffen, z.B. Gewicht oder Größe. Die Ablaufkonfiguration kann dabei beispielsweise aus einer Abfolge aus Anweisungen bestehen, die durch die Steuerung des Magnetresonanztomographen ausführbar sind. Es können auch Ausgaben an eine Bedienperson und Schritte zur Verarbeitung von Eingaben einer Bedienperson enthalten sein. Die Ablaufkonfiguration kann beispielsweise als eine Datei oder eine Mehrzahl zusammengehörender Dateien auf dem Datenspeicher gespeichert sein oder auch in Form einer Datenbank vorliegen. Speichermedien zum Speichern können beispielsweise magnetisch, optischer oder Halbleiterspeicher sein oder auch Speicherplatz in einer Cloud, abstrahiert von dem jeweiligen Speichermedium.

Magnetresonanztomographen können sich, wie bereits dargelegt, in der Hardwareausstattung, beispielsweise in der Anzahl der Empfangs- oder Sendekanäle, der Art und Anzahl von Empfangs- oder Sendeantennen unterscheiden. Es können unterschiedliche Sequenzen in Form von Softwaremodulen zur Ausführung auf dem Magnetresonanztomographen zur Verfügung stehen. Denkbar ist auch, dass sich einzelne Magnetresonanztomographen in der Softwareversion unterscheiden und daher einzelne Anweisungen nicht verstehen oder auf unterschiedliche Art mit unterschiedlichem Ergebnis ausführen. Schließlich wäre es sogar denkbar, dass unterschiedliche Magnetresonanztomographen von unterschiedlichen Herstellern sind.

In dem Schritt, eine Ablaufkonfiguration für den Magnetresonanztomographen in Abhängigkeit von der ersten Systemkonfiguration bereitzustellen, stellt die Konfigurationseinheit mittels der Informationen aus der ersten Systemkonfiguration des über die Identifikation bestimmten Magnetresonanztomographen Schritte bzw. Anweisungen zur Ausführung durch den Magnetresonanztomographen so zusammen, dass diese durch den mittels der Identifikation bestimmten Magnetresonanztomographen dann auch ausführbar sind. Es ist aber auch möglich, dass die Konfigurationseinheit bereits eine Ablaufkonfiguration bereit hält, beispielsweise für bestimmte Aufgaben sortiert, in einem lokalen Speicher, einer Datenbank oder einem Server. Denkbar ist es dabei auch, dass die Konfigurationseinheit einzelne Schritte einer vorhandenen Ablaufkonfiguration je nach Systemkonfiguration in unterschiedliche Anweisungen umsetzt bzw. übersetzt und so auf den durch die Identifikation bestimmten Magnetresonanztomographen anpasst.

Auf vorteilhafte Weise kann das erfindungsgemäße Verfahren sicherstellen, dass eine erstellte Ablaufkonfiguration dann auch ohne nachträgliche Anpassungen oder Änderung auf dem durch die Identifikation bestimmten Magnetresonanztomographen ausführbar ist.

In einer denkbaren Ausführungsform des erfindungsgemäßen Verfahrens weist der Schritt des Bereitstellens einer Ablaufkonfiguration den Schritt auf, mittels der Konfigurationsvorrichtung zu prüfen, ob ein Schritt der Ablaufkonfiguration mit der ersten Systemkonfiguration kompatibel ist.

Wenn beispielsweise die Konfigurationsvorrichtung eine Ablaufkonfiguration für eine bestimmte Aufgabe aus einer Datenbank lädt, so kann das erfindungsgemäße Verfahren auf vorteilhafte Weise feststellen, ob die geladene Ablaufkonfiguration auch für den durch die Identifikation bestimmten Magnetresonanztomographen mit der ersten Systemkonfiguration ausführbar ist.

In einer möglichen Ausführungsform des erfindungsgemäßen Verfahrens weist dieses den Schritt auf, eine Meldung an einen Benutzer der Konfigurationsvorrichtung in Abhängigkeit von einem Ergebnis des Prüfens auszugeben. Das wäre beispielsweise in dem Fall denkbar, dass eine geplante Ablaufkonfiguration auf einem durch die Identifikation bestimmten Magnetresonanztomographen, auch unter den bereits erwähnten Anpassungen, nicht ausführbar ist.

Beispielsweise kann die Konfigurationsvorrichtung beim Prüfen feststellen, dass eine bestimmte Untersuchung nur mit einem mehrkanaligen Sender ausführbar ist. Ist dann der ersten Systemkonfiguration zu entnehmen, dass der durch die Identifikation bestimmte Magnetresonanztomograph nur einen einkanaligen Sender aufweist, so ist eine Ausführung der Untersuchung und einer entsprechenden Ablaufkonfiguration unmöglich. Auf vorteilhafte Weise warnt das erfindungsgemäße Verfahren und die erfindungsgemäße Konfigurationsvorrichtung den Bediener bereits beim Bereitstellen in der Konfigurationsvorrichtung, bevor dann bei einer ersten Anwendung der Ablaufkonfiguration auf dem Magnetresonanztomographen eine Fehlermeldung auftritt und wertvolle Untersuchungszeit verloren geht.

Weitere Vorteile und Einzelheiten ergeben sich aus der nachfolgenden Beschreibung von Ausführungsbeispielen in Verbindung mit den Zeichnungen. Es zeigen in Prinzipdarstellung:
- Fig. 1: eine Ausführungsform eines erfindungsgemäßen Systems mit einem erfindungsgemäßen Magnetresonanztomographen und einer erfindungsgemäßen Konfigurationsvorrichtung;
- Fig. 2: einen Ablaufplan einer Ausführungsform des erfindungsgemäßen Verfahrens.

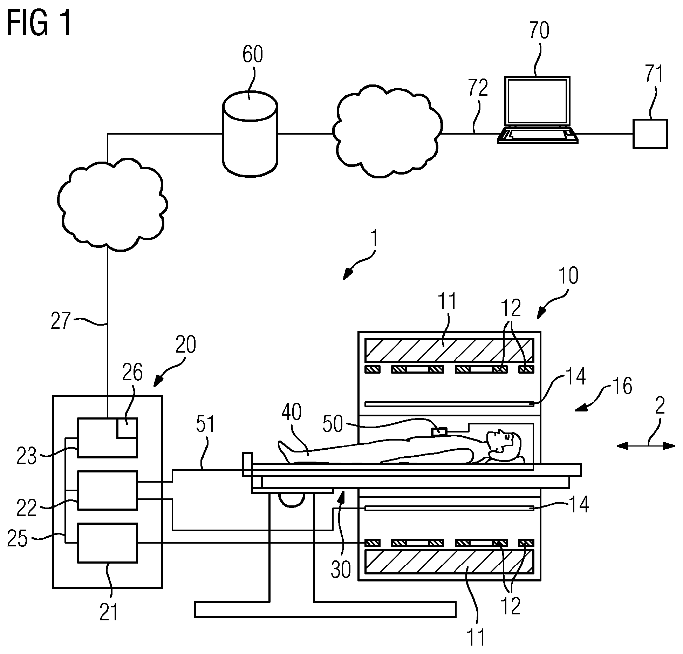

Fig. 1 zeigt eine schematische Darstellung einer Ausführungsform eines erfindungsgemäßen Magnetresonanztomographen 1.

Die Magneteinheit 10 weist einen Feldmagneten 11 auf, der ein statisches Magnetfeld B0 zur Ausrichtung von Kernspins von Proben bzw. in einem Körper eines Patienten 40 in einem Aufnahmebereich erzeugt. Der Aufnahmebereich ist in einem Patiententunnel 16 angeordnet, der sich in einer Längsrichtung 2 durch die Magneteinheit 10 erstreckt. Üblicherweise handelt es sich bei dem Feldmagneten 11 um einen supraleitenden Magneten, der magnetische Felder mit einer magnetischen Flussdichte von bis zu 3T, bei neuesten Geräten sogar darüber, bereitstellen kann. Für geringere Feldstärken können jedoch auch Permanentmagnete oder Elektromagnete mit normalleitenden Spulen Verwendung finden.

Weiterhin weist die Magneteinheit 10 Gradientenspulen 12 auf, die dazu ausgelegt sind, zur räumlichen Differenzierung der erfassten Abbildungsbereiche in dem Untersuchungsvolumen dem Magnetfeld B0 variable Magnetfelder in drei Raumrichtungen zu überlagern. Die Gradientenspulen 12 sind üblicherweise Spulen aus normalleitenden Drähten, die zueinander orthogonale Felder in dem Untersuchungsvolumen erzeugen können.

Die Magneteinheit 10 weist ebenfalls eine Körperspule 14 auf, die dazu ausgelegt ist, ein über eine Signalleitung zugeführtes Hochfrequenzsignal in das Untersuchungsvolumen abzustrahlen und von dem Patient 40 emittierte Resonanzsignale zu empfangen und über eine Signalleitung abzugeben. Weiterhin weist der erfindungsgemäße Magnetresonanztomograph eine oder mehrere Lokalspulen 50 auf, die in dem Patiententunnel 16 nahe am Patient 40 angeordnet sind.

Eine Steuereinheit 20 versorgt die Magneteinheit 10 mit den verschiedenen Signalen für die Gradientenspulen 12 und die Körperspule 14 und wertet die empfangenen Signale aus.

So weist die Steuereinheit 20 eine Gradientenansteuerung 21 auf, die dazu ausgelegt ist, die Gradientenspulen 12 über Zuleitungen mit variablen Strömen zu versorgen, welche zeitlich koordiniert die erwünschten Gradientenfelder in dem Untersuchungsvolumen bereitstellen.

Weiterhin weist die Steuereinheit 20 eine Hochfrequenzeinheit 22 auf, die ausgelegt ist, einen Hochfrequenz-Puls mit einem vorgegebenen zeitlichen Verlauf, Amplitude und spektraler Leistungsverteilung zur Anregung einer Magnetresonanz der Kernspins in dem Patienten 40 zu erzeugen. Dabei können Pulsleistungen im Bereich von Kilowatt erreicht werden. Die einzelnen Einheiten sind über einen Signalbus 25 untereinander verbunden.

Das von der Hochfrequenzeinheit 22 erzeugte Hochfrequenzsignal wird über eine Signalverbindung 31 der Patientenliege 30 zugeführt und an eine oder mehrere Lokalspulen 50 verteilt und in den Körper des Patienten 40 ausgesendet, um dort die Kernspins anzuregen.

Die Lokalspule 50 empfängt dann vorzugsweise ein Magnetresonanzsignal aus dem Körper des Patienten 40, denn aufgrund des geringen Abstandes ist das Signal-zu-Rauschverhältnis (SNR) der Lokalspule 50 besser als bei einem Empfang durch die Körperspule 14. Das von der Lokalspule 50 empfangene MR-Signal wird in der Lokalspule 50 aufbereitet und an die Hochfrequenzeinheit 22 des Magnetresonanztomographen 1 zur Auswertung und Bilderfassung weitergeleitet. Vorzugsweise wird dazu ebenfalls die Signalverbindung 31 genutzt, es sind aber auch separate Signalverbindungen oder eine drahtlose Übertragung denkbar. Es ist ebenso denkbar, dass für das Empfangen eigene Lokalspulen oder andere Antennen vorgesehen sind.

Zur Ausführung des erfindungsgemäßen Verfahrens weist die Steuerung 23 weiterhin eine Ausgabeeinheit 26 auf, mit der die Steuerung 23 eine eindeutige erste Identifikation des Magnetresonanztomographen 1 in einer maschinenlesbaren Form ausgeben kann. Die Ausgabeeinheit 26 kann beispielsweise ein Drucker, ein Display oder eine Schreibeinheit für ein Speichermedium sein.

Darüber hinaus steht die Steuerung 23 über eine erste Datenverbindung 27 mit einem Datenspeicher 60 in Datenverbindung. Die erste Datenverbindung 27 kann beispielsweise ein lokales Netz, ein drahtloses Netz oder eine Datenleitung sein.

Der Datenspeicher 60 ist ausgelegt, eine erste Systemkonfiguration über die erste Datenverbindung 27 von dem Magnetresonanztomographen 1 zu empfangen, zu speichern und die erste Systemkonfiguration mittels der ersten Identifikation eindeutig dem Magnetresonanztomographen 1 zuzuordnen. Als Datenspeicher 60 kommen beispielsweise Server, Netzwerkspeicher oder Datenbanken zur Anwendung. Dabei kann der Datenspeicher auch verteilt in einer Cloud realisiert sein.

Auch eine Konfigurationsvorrichtung 70 steht über eine zweite Datenverbindung 72 mit dem Datenspeicher 60 in Datenaustausch, sodass die Konfigurationseinrichtung 70 auf die erste Systemkonfiguration zugreifen kann. Ansonsten gilt für die zweite Datenverbindung 71 das für die erste Datenverbindung beschriebene.

Die Konfigurationsvorrichtung 70 kann beispielsweise ein Computer mit einem Prozessor, einem Arbeitsspeicher und einem lokalen Datenspeicher zum Speichern von Daten und Programmen sein. Er kann auch ein Laufwerk für Datenmedien aufweisen.

Darüber hinaus weist die Konfigurationsvorrichtung 70 eine Eingabeeinheit 71 auf, mit der die Konfigurationsvorrichtung 70 eine erste Identifikation erfassen kann. Dabei kann die Eingabeeinheit 71 je nach Medium für die erste Identifikation unterschiedlich ausgeführt sein. Ist die erste Identifikation beispielsweise als Barcode oder QR-Code ausgeführt, so kann die Eingabeeinheit 71 eine Kamera oder Scanner sein. Dabei ist es beispielsweise auch denkbar, dass die Kamera die erste Identifikation direkt von einem Display der Ausgabeeinheit 26 des Magnetresonanztomographen 1 ausliest, beispielsweise wenn die Konfigurationsvorrichtung 70 als tragbarer Computer ausgeführt ist. Eine Kamera oder ein Scanner kann aber ebenso eine erste Identifikation auf einem Papier erfassen. Ebenso sind aber auch beliebige Datenträger denkbar, um die erste Identifikation zu der Konfigurationsvorrichtung 70 zu übertragen. Selbst eine drahtlose Verbindung über WLAN, Bluetooth oder Near Field Communication wäre denkbar, um die erste Identifikation von dem Magnetresonanztomographen 1 zu einer mobilen Konfigurationsvorrichtung 70 zu übertragen.

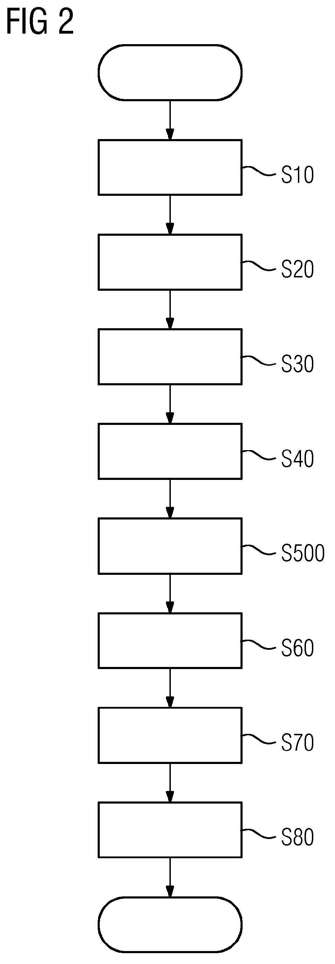

Die Fig. 2 zeigt einen schematischen Ablaufplan einer Ausführungsform des erfindungsgemäßen Verfahrens. Die Nummerierung der Schritte S10 bis S80 entspricht dabei nicht notwendigerweise der Reihenfolge der Ausführung.

In einem Schritt S10 des Verfahrens speichert der Magnetresonanztomograph 1, vorzugsweise die Steuerung 23, eine erste Systemkonfiguration des Magnetresonanztomographen 1 auf dem Datenspeicher 60. Das Speichern erfolgt über die erste Datenverbindung 27. Der Datenspeicher 60 kann beispielsweise ein Server oder eine Cloud sein und zur Übertragung der Systemkonfiguration ein Übertragungsprotokoll wie FTP oder SMB verwendet werden. Die erste Systemkonfiguration kann dabei beispielsweise in einem lokalen Speicher der Steuerung 23 abgelegt sein oder vor der Übertragung von der Steuerung 23 aus einzelnen gespeicherten Informationen zusammengestellt werden.

Vorzugsweise ist die erste Systemkonfiguration mit einer eindeutigen Kennzeichnung versehen, sodass sie von anderen Systemkonfigurationen anderer Magnetresonanztomographen unterschieden werden kann. Denkbar ist es beispielsweise, dass die erste Systemkonfiguration einen eindeutigen Dateinamen erhält oder ein Datenfeld für eine Identifikation vorgesehen ist. In einem Schritt S20 erzeugt die Steuerung 23 des Magnetresonanztomographen 1 eine Identifikation der ersten Systemkonfiguration. Diese Identifikation kann dann beispielsweise als Bestandteil des Dateinamens oder der ersten Systemkonfiguration verwendet werden.

In einem Schritt S30 gibt die Steuerung 23 die Identifikation über die Ausgabeeinheit 26 des Magnetresonanztomographen 1 aus. Vorzugsweise erfolgt die Ausgabe in einer Form, die durch eine andere maschinelle Vorrichtung erfasst werden kann, um Fehler bei der Übertragung durch einen Bediener zu vermeiden. Denkbar ist beispielsweise die Kodierung der Identifikation in einem binären Barcode oder einem QR-code, der über einen Drucker als Ausgabeeinheit 26 ausgegeben wird. Ein derartiger Code könnte aber auch über ein Display ausgegeben und von einem Bediener z.B. mit einem Smartphone fotografiert werden. Denkbar ist auch die Ausgabe der Identifikation auf einem maschinenlesbaren Speicher wie einem USB-Stick. Umgekehrt wäre aber auch die Ausgabe einer Identifikation als Zeichenkette auf einem Display oder Drucker möglich, sodass der Bediener die Identifikation beispielsweise abschreibt oder zumindest später manuell über eine Tastatur eingibt.

In einem Schritt S40 erfasst die Eingabeeinheit 71 der Konfigurationsvorrichtung 70 die Identifikation. Beispielsweise kann die Konfigurationsvorrichtung 70 ein mobiler Computer mit einer Kamera als Eingabeeinheit 70 sein, sodass die Kamera den Barcode oder den QR-Code direkt erfasst und Konfigurationseinrichtung 70 daraus die Identifikation mit maschinellen Erkennungsalgorithmen bestimmt. Die Eingabeeinheit 71 kann aber auch ein Lesegerät für Datenspeicher sein, sodass die Identifikation z.B. auf einem USB-Stick von der Konfigurationsvorrichtung 70 ausgelesen werden kann. Ebenso ist es denkbar, dass der Bediener eine in Textform ausgedruckte Identifikation über eine Tastatur als Eingabeeinheit 71 in die Konfigurationsvorrichtung 70 eingibt.

In einem Schritt S50 greift die Konfigurationsvorrichtung 70 auf die erste Systemkonfiguration in dem Datenspeicher 60 über die zweite Datenverbindung 72 mithilfe der Identifikation zu. Beispielsweise kann die Identifikation ein eindeutiger Dateiname sein, sodass die Konfigurationsvorrichtung 70 die erste Systemkonfiguration über ein Datenübertragungsprotokoll wie FTP oder SMB in einen internen Speicher laden kann. Es ist aber auch denkbar, dass die Identifikation ein Schlüssel ist, über den die Konfigurationsvorrichtung Zugang auf einzelne Informationen der ersten Systemkonfiguration in einer Datenbank als Speicher 60 erhält.

In einem Schritt S60 stellt die Konfigurationsvorrichtung eine Ablaufkonfiguration für den Magnetresonanztomographen 1 in Abhängigkeit von der ersten Systemkonfiguration bereit. Beispielsweise kann die Konfigurationsvorrichtung 70 aus der ersten Systemkonfiguration die Softwareversion einer Betriebssoftware des Magnetresonanztomographen 1 entnehmen und nur der Softwareversion entsprechende Anweisungen in der Ablaufkonfiguration verwenden. Gleiches gilt für die Hardwareausstattung, beispielsweise muss ohne ein entsprechend großes Lokalspulenarray ein Ganzkörperscan möglicherweise öfter unterbrochen werden, um die Lokalspule 50 zu repositionieren.

In einem Schritt S70 prüft die Konfigurationsvorrichtung 70, ob ein Schritt der Ablaufkonfiguration mit der ersten Systemkonfiguration kompatibel ist. Es ist beispielsweise denkbar, dass die Syntax der verwendeten Anweisungen mit der Softwareversion der Betriebssoftware verglichen wird. Dass kann insbesondere relevant sein, wenn die Ablaufkonfiguration beispielsweise nicht in Schritt S60 neu erzeugt wird, sondern von einem anderen Magnetresonanztomographen 1 übernommen werden soll.

In einem anderen Schritt S80 gibt die Konfigurationsvorrichtung in Abhängigkeit von einem Ergebnis des Prüfens in Schritt S70 Meldung an einen Benutzer aus. Beispielsweise kann die Konfigurationsvorrichtung 70 eine Warnung ausgeben, dass die Ablaufkonfiguration auf dem Magnetresonanztomographen 1 nicht ausgeführt werden kann. Die Konfigurationsvorrichtung 70 kann dazu auch einen möglichen Grund und/ oder Abhilfemaßnahmen ausgeben. Beispielsweise kann die Meldung den Hinweis beinhalten, dass die Softwareversion nicht kompatibel ist und ein Softwareupgrade bei dem Magnetresonanztomographen 1 erforderlich ist, oder dass zusätzliche Hardware nachgerüstet werden muss, um z.B. eine Multislice-Sequenz ausführen zu können.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Magnetresonanztomograph mit einer Steuerung (23) und einer Ausgabeeinheit (26),
wobei die Steuerung (23) des Magnetresonanztomographen (1) ausgelegt ist, eine erste Systemkonfiguration in einem Datenspeicher (60) für eine Mehrzahl an Systemkonfigurationen über eine erste Datenverbindung (27) abzuspeichern,
wobei die Steuerung (23) des Magnetresonanztomographen (1) ausgelegt ist, eine Identifikation für die erste Systemkonfiguration auf einem maschinenlesbaren Medium mittels der Ausgabeeinheit (26) auszugeben.

2. System zum Abgleich von Systemkonfigurationen für einen Magnetresonanztomographen (1), wobei das System aufweist:
einen Magnetresonanztomographen (1) nach Anspruch 1,
eine Konfigurationsvorrichtung (70) mit einer Steuerung und
einer Eingabeeinheit (71),
einen Datenspeicher (60), wobei der Datenspeicher (60) ausgelegt ist eine Mehrzahl an Systemkonfigurationen für eine Mehrzahl an unterschiedlichen Magnetresonanztomographen zu speichern,
und eine zweite Datenverbindung (72) zwischen Konfigurationsvorrichtung (70) und Datenspeicher (60) sowie die erste Datenverbindung (27) zwischen Datenspeicher (60) und Magnetresonanztomograph (1),
wobei die Steuerung der Konfigurationsvorrichtung (70) ausgelegt ist, die Identifikation für die erste Systemkonfiguration von dem maschinenlesbaren Medium zu erfassen.

3. System nach Anspruch 2, wobei die Konfigurationsvorrichtung (70) ausgelegt ist, anhand der Identifikation die erste Systemkonfiguration in dem Datenspeicher (60) zu identifizieren und in Abhängigkeit von der ersten Systemkonfiguration eine Ablaufkonfiguration für den Magnetresonanztomographen (1) bereitzustellen, der die Identifikation ausgegeben hat.

4. System nach Anspruch 3, wobei die Ausgabeeinheit (26) ausgelegt ist einen QR-Code mit der Identifikation bereitzustellen und die Eingabeeinheit (71) ausgelegt ist, den QR-Code zu erfassen.

5. Verfahren zum Abgleich einer Systemkonfiguration eines Magnetresonanztomographen (1) mit einer Konfigurationsvorrichtung (70) mittels eines Datenspeichers (60),
wobei der Magnetresonanztomograph (1) eine Steuerung (23) und eine Ausgabeeinheit (26) aufweist,
wobei die Konfigurationsvorrichtung (70) eine Steuerung und eine Eingabeeinheit (71) aufweist,
wobei eine zweite Datenverbindung (72) zwischen Konfigurationsvorrichtung (70) und Datenspeicher (60) sowie eine erste Datenverbindung (27) zwischen Datenspeicher (60) und Magnetresonanztomograph (1) besteht,
und das Verfahren die Schritte aufweist:
(S10) Speichern einer ersten Systemkonfiguration des Magnetresonanztomographen (1) auf dem Datenspeicher (60) durch die Steuerung (23) des Magnetresonanztomographen (1);
(S20) Erzeugen einer Identifikation der ersten Systemkonfiguration durch die Steuerung (23) des Magnetresonanztomographen (1) ;
(S30) Ausgeben der Identifikation über die Ausgabeeinheit (26) des Magnetresonanztomographen (1);
(S40) Erfassen der Identifikation durch die Eingabeeinheit (71) der Konfigurationsvorrichtung (70);
(S50) Zugreifen auf die erste Systemkonfiguration in dem Datenspeicher (60) durch die Konfigurationsvorrichtung über die Datenverbindung mithilfe der Identifikation.

6. Verfahren nach Anspruch 5, wobei das Verfahren weiterhin den Schritt aufweist:
(S60) Bereitstellen einer Ablaufkonfiguration für den Magnetresonanztomographen (1) in Abhängigkeit von der ersten Systemkonfiguration.

7. Verfahren nach Anspruch 6, wobei bei der Schritt des Bereitstellens einer Ablaufkonfiguration die Schritte aufweist:
(S70) Prüfen durch die Konfigurationsvorrichtung (70), ob ein Schritt der Ablaufkonfiguration mit der ersten Systemkonfiguration kompatibel ist.

8. Verfahren nach Anspruch 7, wobei das Verfahren weiterhin den Schritt aufweist:
(S80) Ausgeben einer Meldung an einen Benutzer der Konfigurationsvorrichtung (70) in Abhängigkeit von einem Ergebnis des Prüfens.

9. Computerprogrammprodukt, welches direkt in einen Prozessor einer programmierbaren Steuerung ladbar ist, mit Programmcode-Mitteln, um alle Schritte eines Verfahrens nach einem der Ansprüche 5 bis 8 auszuführen, wenn das Programmprodukt auf der Steuerung ausgeführt wird.

10. Computerlesbares Speichermedium mit darauf gespeicherten elektronisch lesbaren Steuerinformationen, welche derart ausgestaltet sind, dass sie bei Verwendung des Speichermediums in einer Steuerung eines Magnetresonanztomographen (1) nach Anspruch 1 das Verfahren nach einem der Ansprüche 5 bis 8 durchführen.
